# EUROPEAN PATENT SPECIFICATION

(11) **EP 4 399 519 B1**
(45) Date of publication and mention of the grant of the patent: **08.07.2026**
(21) Application number: 22776991.6
(22) Date of filing: 24.08.2022
(51) Int. Cl.: G01N 33/24

(54) **INSTANT EVAPORIMETER FOR GRASSY TURF**
SOFORT-EVAPORIMETER FÜR GRASFLÄCHEN
EVAPORIMÈTRE INSTANTANÉ POUR GAZON HERBEUX

(30) Priority: 06.09.2021 IT 202100022955
(43) Date of publication of application: 17.07.2024
(73) Proprietor: Università Cattolica del Sacro Cuore, 20123 Milano (IT)
(72) Inventor: PONI, Stefano, 00168 Roma RM (IT); MAGNANINI, Eugenio, 00168 Roma RM (IT)
(74) Representative: Manna, Sara
(86) International application number: PCT/IB2022/057918
(87) International publication number: WO 2023/031734

(56) References cited:
- WO-A1-2019/213708
- CN-B- 107 290 507
- CN-U- 209 570 479
- US-A1- 2013 235 378
- MCLEOD MALEM K ET AL: "Evaluation of an enclosed portable chamber to measure crop and pasture actual evapotranspiration at small scale", AGRICULTURAL WATER MANAGEMENT, vol. 67, no. 1, 1 June 2004 (2004-06-01), NL, pages 15 - 34, XP055914715, ISSN: 0378-3774, DOI: 10.1016/j.agwat.2003.12.006

## Description

### Technical field of the invention

The present invention relates to a device for measuring the evaporation/transpiration parameters from a portion of bare soil or soil covered by vegetation.

### Background

By specifically, but not exclusively, referring to orchard and vineyard ecosystems and under the pressure of an increasingly eco-sustainable agricultural management of crops, the use of spontaneous or artificial, temporary or permanent, total or partial grassing (cover crops) is attracting considerable interest, acting as alternative to the use of herbicides and traditional processing. However, the insertion a cover crop element in an ecosystem raises important critical issues, particularly from the point of view of the water and nutritional competition against the associated primary crop, with particular relevance in the high-income tree systems. Such water competition assumes a particular relevance in an era in which the global warming is leading to a worsening of the seasonal water balance. Therefore, it is essential to succeed in measuring, quickly and precisely, the water consumption rate of the cover crops with the purpose of:
a) quantifying the (daily or seasonal) extent of water loss amount directly attributable to the grass transpiration;
b) calibrating the irrigation water return depending upon the above-mentioned amount.

The raised problems become even more complex depending upon, both spatial and temporal, variability of the water consumption parameter.

As far as spatial variability is concerned, it is known in fact that in the soil - especially depending upon its heterogeneity, which even within minimum surfaces (for example < 1 ha) may result to be high - areas create which, due to differences in texture, organic substance, porosity, water infiltration capability and compaction resistance, can inhibit or favour the cover crop growth, by conditioning directly even the water consumption extent. Moreover, it is also known that cover crop has a high seasonal variability of its own water consumption depending upon several factors, such as: climatic trend, growth rates, mowing periods and relative post-mowing re-growth, temporary summer withering with possible autumn recovery. The mentioned elements emphasize the need for being able to perform, in various moments of the day or on different dates during the season, quick measurements of the soil water loss extent, with possibility of moving quickly from one point to another of the plot.

As it is known, the estimate of the hourly, daily or seasonal water loss of a bare soil or soil covered by cover crop is typically entrusted to a reference method, providing the use of a class-A evaporimeter. It is a circular tank of standard sizes (1220 mm diameter and 254 mm height) which can be positioned on a uniform, continuous and low-cut cover crop. The tank contains water and, through an inspection well, allows to detect periodically the lowering level of the same by means of a micrometric screw or an electrical sensor. Alternative instruments for measuring the soil evaporation rate, or evapotranspiration from a surface invested with one or more vegetable essences, consists of various types of atmometers (Bellani, Livingston, Piche etc..). They mainly consist of an approximately 30-cm-high graduated cylindrical container which, through a suction pipe, feeds an evaporating surface made of a porous ceramic disk; the device in this case is placed at about 100-120 cm above the soil level.

A third alternative known in the art is represented by the tilting weighing lysimeters which, depending upon sizes - always quite bulky - provide a 1-2-meter-high cylinder made of metal or plastic containing a representative sample of soil, embedded in the soil, which can be periodically lifted, weighed and put back into place.

Centinari et al. [Centinari, M., Filippetti, I., Bauerle, T., Allegro, G., Valentini, G., Poni, S., 2013. Cover crop water use in relation to vineyard floor management practices. American Journal of Enology and Viticulture 64, 522-526*. https:*//*doi.org*/*10.5344*/*ajev.2013.13025*] proposed and tested a miniaturized version of lysimeter (mini-lysimeter) in the vineyard ecosystem mainly consisting of a pot with variable volume, embedded in the soil so as to avoid projections with respect to the ground level. The pot can be left with bare soil or soil grassed with the same essences surrounding it. Lifting, weighing and repositioning of the pot (all manual procedures which can be performed at various intervals) allow to obtain a direct estimation of the water amount lost in the time unit.

Finally, one last approach known in the art is the one referring to the use of quite expensive portable systems for gas exchanges with which, by using a leaf chamber usually of cylindrical type, it is possible to perform a direct measurement of transpiration of a modest portion of cover crop.

As alternative, the work published in 2009 by Centinari et al. *[*Centinari, M., Poni, S., Filippetti, I., Magnanini, E., Intrieri, C., 2009. Evaluation of an open portable chamber system for measuring cover crop water use in a vineyard and comparison with a mini-lysimeter approach. Agricultural and Forest Meteorology 149, 1975-1982. https://doi.org/10.1016/j.agrformet.2009.07.005] proposed the use of a cylindrical chamber for measuring gas exchanges (potentially transpiration, photosynthesis and respiration) of soil portions. The proposed system, however, is of open type, that is it requires a continuous flow of air, generated by a fan or external pump, circulating through the chamber. The measurement of the gas exchanges - determined as difference in the concentration of water vapor and carbon dioxide entering and exiting the chamber - takes place by using an infrared gas analyser (IRGA). The instrument has a surface resting onto the soil of 2800 cm², a height of 70 cm and a volume of 0.196 m³ and it was implemented by composing self-built elements (for example the chamber itself and the inlet and outlet air conveyance duct) and commercial components (IRGA and flow dispensing pump) with obvious cost increase. However, the system of "open" type does not make such device suitable to quick measurements, repeated over time, in different points of the soil, since the flow adjustment depending upon the specific measurement conditions (for example bare soil or soil with highly variable development cover crops) is an unavoidable and difficult step.

US2013235378 discloses a known system for determining flux of a component of a gas from soil comprising a chamber chamber and a lid defining a first cavity, the chamber walls defining an opening for sealably contacting the cavity with a soil location, the chamber walls further defining one or more outlet openings between the cavity and atmosphere surrounding the chamber; the chamber further comprising an inlet membrane covering the opening having an inlet membrane and one or more first outlet membranes covering the one or more first outlet openings having a lower diffusivity than the inlet membrane diffusivity; and one or more measuring devices for measuring a first concentration of first isotopologue of the gas of interest.

In such context, then it appears urgent the need for having available more effective solutions for determining the rate of water consumption of cover crops which could conjugate, in particular, a better handling and portability to optimum accuracy and timeliness.

### Summary of the invention

The technical problem underlying the present invention is then to provide a device for measuring the evaporation and/or transpiration parameters from a portion of bare soil or soil covered by vegetation, allowing to meet the needs mentioned above with reference to the known art.

Such problem is solved by a device according to claim 1.

Preferred features of the present invention are set forth in the depending claims.

The invention provides a device that, in its preferred meaning, mainly comprises:
▪ a main body extending from an open upper end to an open lower end, opposed to one another, which main body defines a gas passage chamber;
▪ a closing element adapted to sealingly insert in the open upper end, so as to seal on the upper side the main body during the measurement step; the closing element comprises detection means (transducers) configured to measure the evaporation and/or transpiration parameters, which are associated to the closing element and are facing the inside of the main body when the closing element is inserted in the upper end of the main body; and
▪ anchoring devices configured to sealingly associate to the lower end of the main body and to penetrate at least partially the soil portion, so as to seal on the lower side the main body and to immobilize the device itself to the soil during the measurement step.

Preferably, the anchoring means used to immobilize the invention device to the soil consists of a substantially truncated-cone shaped body provided with a sharp or tapered outer end, suitable to penetrate the soil portion. Advantageously, the anchoring means can be also provided with manipulation means, for example in the form of handles, suitable to ease the procedures for positioning and pushing the anchoring means into the soil.

The insertion of the closing element in the upper end of the main body of the herein described device, when the device is immobilized to the soil by the anchoring means, allows to define the chamber as pneumatic sealing "closed" system, delimited on the lower side by the soil surface. The generation of such "closed" system allows to remove the problems related to the generation of a continuous air flow entering and exiting the chamber during the step of measuring the evaporation and/or transpiration parameters and, therewith, the need for providing itself with a flow dispensing pump powered by alternating current. Consequently, the device the invention relates to allows to reach a *steady state* regime in no more than 120 seconds, thus allowing to perform a very high number of measurements in short time.

Therefore, the device, in its various embodiments, allows to obtain an improvement in determining the evaporation and/or transpiration parameters, in particular of the evapotranspiration parameters, as well as of the rate of water consumption, both from bare soil and soil covered partially or totally by vegetation (for example grass, cover crop), particularly in terms of accuracy and rapidity.

The simplification offered by the device the invention relates to allows to remove two important problems which can be commonly found in the devices conventionally used in the field, that is:
i) the need for an accurate measurement of the flow which can be used directly in calculating the transpiration rates;
ii) a preventive calibration of the flow depending upon the expected transpiration rates.

In this case, an excessive flow with respect to the amount of leaf surface enclosed in the chamber can determine a poor sensitivity of the instrument in recording minimum transpiration variation; vice versa a too low flow can easily lead to problems of excessive overheating of the air present in the chamber, with significant alterations of the vapor pressure deficit (VPD) of the air surrounding the vegetable covering until causing, in particularly hot days, leaf damages due to burn and subsequent necrosis.

Embodiments of the device the invention relates to advantageously result to be not very sensitive to problems of air overheating inside the chamber for two main reasons: 1) measuring time does not exceed 60-80 seconds; 2) the progressive build-up of relative humidity (RH) inside the "closed" chamber contributes to calm the overheating effect.

According to preferred aspects, the device developed by the authors of the present invention results to be agile and light and can be easily and quickly moved in various areas of the field by acquiring direct transpiration measurements in very short periods of time. Such measurements can also be displayed, for control needs, on an outer display and subsequently stored in a local memory. The optional possibility of recording the obtained data on SD-card results to be particular important, as well as the possibility of accessing the raw data even through serial communication with the device, by means of suitable data transmission cable, available on mobile phones with Android operating system.

The device, then, thanks to inherent characteristics of overall dimension, weight and principle of use, guarantees a definitely advantageous portability and implementation with respect to the conventional devices described in the prior art.

Moreover, the device provides the use of components with low or very low cost and it has sizes and weight so as to allow a quick transportation and re-positioning thereof in the subsequent point of measurement.

Thanks to the particular favourable combination between purchase costs and measurement rapidity/accuracy, both on bare and grassy soil, the device the present invention relates to is extremely competitive, and it can be easily used for scientific, experimental, certification, calibration or span purposes.

The device can be used for example by companies and firms working in the "green" field for which it is necessary to acquire, quickly but at the same time with good precision, data on water consumptions of the cover crops which are proposed or, alternatively, by companies and firms engaged in the seed sector (for example production of pure or mixed mixtures for artificial grassing) for which it is necessary to acquire basic data useful to quantify the water consumption rate thereof and, then, the potential competitive action against other possibly associated species.

Additional advantages, features and use modes of the present invention will result evident from the following detailed description of some embodiments, shown by way of example and not for limiting purposes.

### Brief description of figures

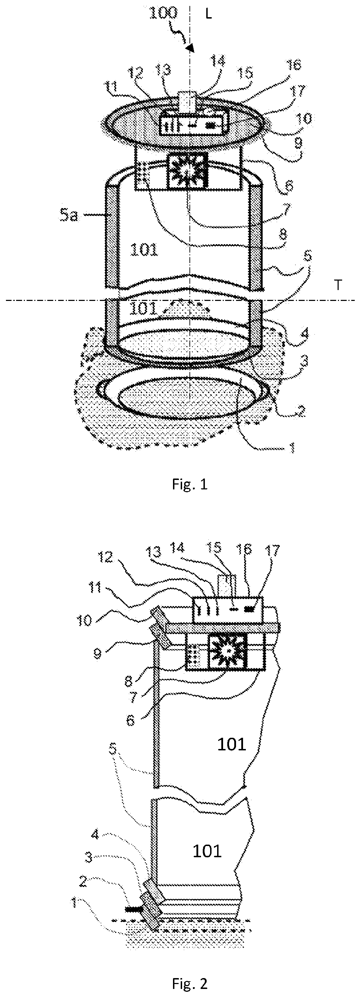

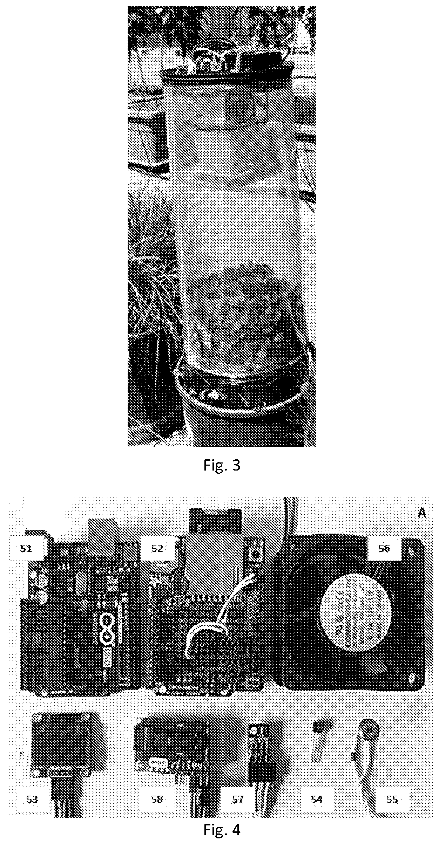

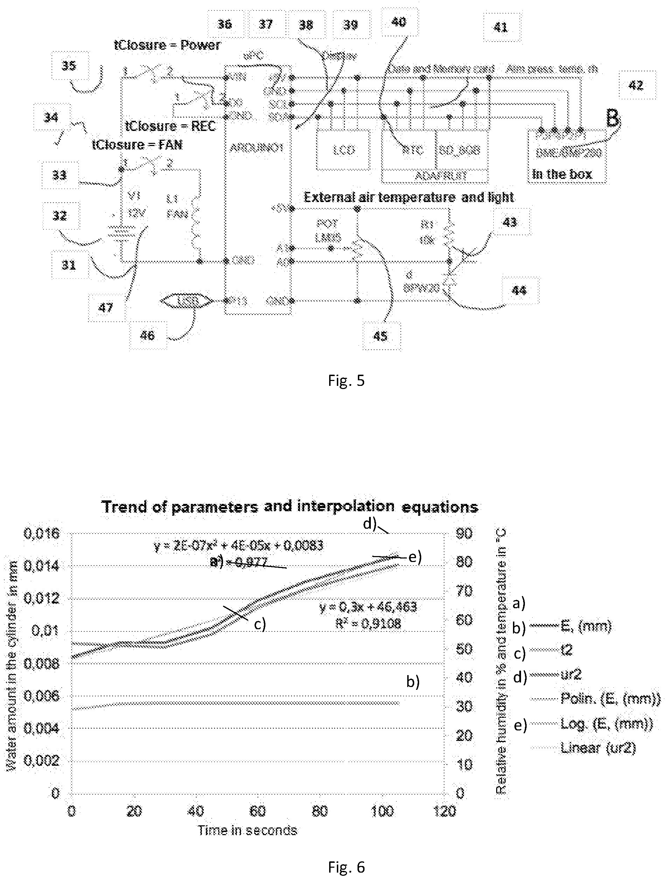

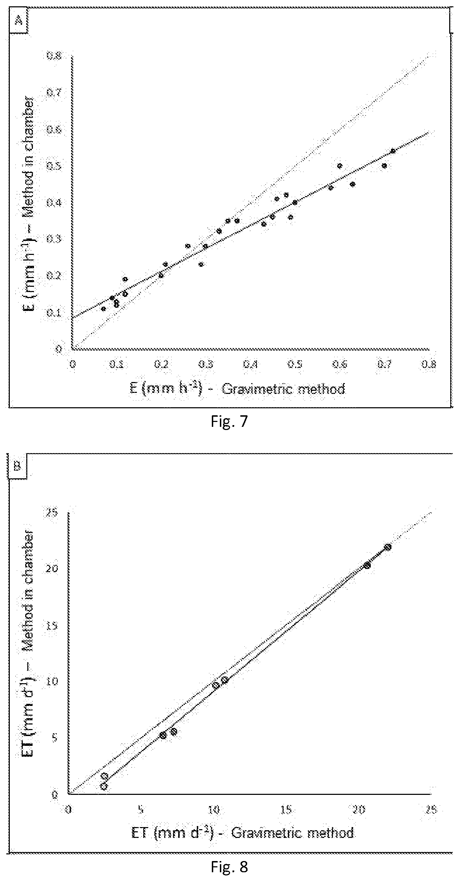

Reference will be made to the figures of the enclosed drawings, wherein:
▪ Figure 1 shows schematically a partially section side perspective view of a first embodiment of a device according to the invention;
▪ Figure 2 shows schematically a side section view of the device of Figure 1;
▪ Figure 3 shows a side perspective view of an additional preferred embodiment of a device according to the invention;
▪ Figure 4 shows in detail some components of a still additional preferred embodiment of a device according to the invention;
▪ Figure 5 shows a series of logic components of a preferred embodiment of a device according to the invention;
▪ Figure 6 shows a graph illustrating the trend of parameters and interpolation equations related to a preferred embodiment of a device according to the invention;
▪ Figure 7 shows on a comparative basis the linear regression between the water loss by evaporation from the wet cloth (hourly rates) measured gravimetrically and by means of a preferred embodiment of a device according to the invention;
▪ Figure 8 shows on a comparative basis the daily water loss (mm/days) measured by weighing and by means of a preferred embodiment of a device according to the invention.

In the above-mentioned figures, the sizes are to be meant as purely exemplifying and not necessarily with components shown in proportion.

### Detailed description of preferred embodiments

Different embodiments and variants of the invention and portions thereof will be described hereinafter, based upon different aspects thereof usable separately or in combination.

Analogous components are designated in the different Figures with the same reference number.

In the following detailed description additional embodiments and variants with respect to embodiments and variants already treated in the same description will be illustrated limitedly to the differences with what already illustrated.

Moreover, as said, the different embodiments and variants described hereinafter are likely to be used in combination.

By firstly referring to Figure 1, a device according to a preferred embodiment of the invention is designated as a whole with 100.

The device 100 is configured for measuring the evaporation and/or transpiration parameters from a soil portion which can be bare or covered by vegetation. Under the expression "bare soil", in the context of the present invention, a soil portion without vegetation is meant. Under the expression "soil covered by vegetation", in the context of the present invention, a soil portion partially or totally covered by vegetation, for example by grass or plants, is meant. The expression "soil covered by vegetation", for example, comprises a cover crop, that is the set of graminaceous plants and/or other grasses forming a continuous carpet and which can provide food for grazing animals or perform functions not linked to the agricultural and environmental productions, such as for example landscape and recreational function.

According to a preferred embodiment of the invention, the device 100 in particular allows to obtain a measurement of the evapotranspiration parameters from a soil portion. Under the term "evapotranspiration", also known with abbreviation "ET", in the context of the present invention, the amount of water (referred to the time unit) is meant that from the soil passes into air at the vapor status due to the combined effect of transpiration through plants and of evaporation directly from the soil.

To this purpose, the device 100 comprises: a main hollow body 5 which defines a measuring chamber 101 suitable to abut on the considered soil portion; a closing element 10 of an upper end of the main body 5; and means 1 anchoring to the soil of the main body 5.

Preferably, the shape of the main body 5 is elongated, with a preferential development along a longitudinal direction L which results to be vertical in use, in particular orthogonal to the external surface of the soil portion to be analysed. The main body 5 comprises one or more side walls 5a extending between an open upper end and an open lower end of the main body 5, the latter being opposed to one another along such longitudinal direction L.

In particular, the main body 5 can consist exclusively of one or more side walls 5a.

The shape of the side walls 5A is preferably cylindrical to implement a tubular main body 5, as in the preferred embodiment shown in Figure 1. Still more preferably, the section of the main body 5 is constant along the entire length of the main body according to the longitudinal direction L, and in particular it has circular geometry. According to an additional aspect of the invention, the side walls 5a of the main body 5 have a thickness comprised between 0.75 and 2 mm, preferably equal to 0.75 mm.

However, additional variants of the device 100 can include a main body with prismatic shape, even with variable section along the longitudinal direction L.

The main body 5 can have variable length and width along the longitudinal direction L or transversal direction T.

According to a preferred embodiment, the main body has a height comprised between 456 and 684 mm, preferably equal to 570 mm, and/or a surface resting onto the soil comprised between 393 and 589, preferably equal to 491 cm².

Under the expression "surface resting onto the soil", in the context of the present description, the area of the open lower terminal section of the main body 5 is meant, then the investigated soil area, which can correspond to the section of the measurement chamber 101 when the main body 5 has constant section.

The main body 5 of the device according to any one of the herein described embodiments, defines, when it is in closed configuration, an internal volume - corresponding to the volume of the measurement chamber - comprised between 22 and 34, preferably equal to 28 I.

According to a preferred embodiment, said main body 5 is made of transparent, preferably plastic, material. Not limiting examples of materials that can be used to implement the main body 5 include polyethylene, polymethyl methacrylate, Teflon, mylar.

Still according to a preferred embodiment, the open lower end of the main body 5 has a substantially tapered or truncated-cone shape to implement a sealingly connection with the above-mentioned anchoring means 1.

The side walls 5a of the main body 5 internally define the measuring chamber 101, also defined as gas passage chamber.

As it will be explained in greater details hereinafter, the main body 5 is configured to allow the collection and passage of gases exchanged from the soil to the external environment, in particular carbon dioxide and water vapor passing into the air from soil due to the effect of the transpiration through the plants, or of the water evaporation directly from the soil.

The device 100 further comprises the closing element 10, shaped as a plug configured to sealingly couple with the open upper end of the main body 5, so as to seal on the upper side the main body 5 during the measurement step. To this purpose, a threaded connection for example can be provided, and a gasket can be interposed between the extremal edges of the upper end of the main body 5 and of the closing element 10. According to the preferred embodiment of the invention shown in Figure 1, even the section of the closing element 10 is circular.

The closing element 10 preferably has a disc-like flat shape and it bears a mutually opposed upper surface and lower surface. The closing element 10 comprises detection means 8 configured for measuring the evaporation and/or transpiration parameters of the soil portion. The expression "detection means", used in the context of the present invention, relates to one or more sensors suitable to measure evaporation and/or transpiration parameters in the passage chamber 101. The parameters can include one or more of the following ones: partial pressure of vapor, temperature, relative humidity, carbon dioxide concentration, atmospheric pressure.

To this purpose, the closing element 10 can bear dedicated casings or at least it can be provided with means for connecting with the detection means 8. The latter are associated to the closing element 10 at its internal surface, so that, during use, they are facing the inside of the main body 5.

According to a preferred embodiment of the invention, the closing element 10 can be configured to implement a "head" having a substantially truncated-cone shape, with concavity facing the main body 5, and it is preferably made of plastic material. The material used to implement the above-mentioned closing element can be coated externally with a joint or gasket material, for example with sponge, that is a material which is capable of guaranteeing, when said closing means 10 is inserted in the upper end of the main body 5 of the device 100, an optimum pneumatic sealing between the two components.

The device 100 further comprises the already mentioned anchoring means 1, configured to engage sealingly, in particular by penetrating, at least partially, the soil portion to be analysed. Such anchoring means 1 is coupled at its own proximal terminal end thereof to the lower end of the main body 5, whereas at its own distal terminal end thereof it is configured to penetrate at least partially the soil portion. In particular, the anchoring means 10 is configured to seal on the lower side the main body 5, and then the measurement chamber 101, with respect to the soil portion to be analysed as well as to immobilize the device 100 to the soil during the step of measuring the evaporation and/or transpiration parameters.

According to the present invention, during the step of measuring the evaporation and/or transpiration parameters from said soil portion, the device 100 has a configuration showing (i) the sealingly coupling of the anchoring means 1 to the lower end of said main body 5, (ii) the insertion of the anchoring means in the soil so as to immobilize the main body 5 to the soil and (iii) the sealingly coupling of said closing element 10 with the upper end of the main body 5. In such configuration, the main body 5 of the above-mentioned device 100 then defines a gas passage internal chamber 101 delimited on the lower side by the soil surface and delimitated on the upper side by said closing element 10.

In a preferred embodiment variant, the anchoring means 1 of the device 100 according to the invention comprises or consists of a hollow body having a substantially truncated-cone shape with convexity facing the main body 5, preferably provided with a sharp or tapered external edge suitable to penetrate the soil portion.

Preferably, to facilitate the procedures for installing the device 100, the anchoring means 1 is provided with manipulation means 2, for example in form of two handles arranged laterally on the external surface of the anchoring means 1, in a portion proximal to the main body 5 and intended to remain above ground.

According to an additional preferred embodiment, the anchoring means 1 comprises or consists of a metal ring, preferably made of aluminium. The ring has a substantially truncated-cone shape with convexity facing the main body 5. The free terminal edge of the ring is sharp or cutting to allow, by means of a rotation procedure eased by the presence of the possible side handles and a downward push, the penetration for few centimetres (preferably about 3) of the ring itself in the soil.

Advantageously, the anchoring means 1 according to any one of the previously described embodiments, for example in the form of metal ring, can be constrained manually at the lower end of the main body 5 and, thanks to the above-mentioned concavity, is capable of compressing the soil edge by creating a good gas tightness both externally and internally, by preventing the outgoing thereof from the main body 5.

The device 100 according to any one of the herein described embodiments can further comprise at least a first junction element 9 interposed between the closing element 10 and the upper end of the main body 5. Moreover, at least a second junction element 3 can be interposed between the anchoring means 1 and said lower end of the main body 5.

The presence of said one or more junction elements 3, 9 allows to guarantee or improve the pneumatic sealing of the device 100 during the measuring step. Preferably, the junction elements 3 or 9 consist of a material suitable to guarantee the pneumatic sealing between the different components of the device. A not limiting example which can be used to implement the above-mentioned junction elements is represented by sponge with closed cells, for example in form of tape. The spongy junctions represent an optimum solution since they offer a good sealing and, if they deteriorate, it is simple to replace them.

The junction elements, however, can consist of other materials known in the art, provided that they are suitable to guarantee an optimum pneumatic sealing of the device 100 during the measuring step.

As mentioned previously, during the measurement step, the main body 5 of the device according to the invention defines an internal chamber 100 delimited on the lower side by the surface of the soil portion under examination and sealed on the upper portion by said closing element 10. Such configuration advantageously allows to generate a system of "closed" type, which prevents the circulation of external air.

By purely way of example, the main body 5 of the device 100 according to the invention can be rested upon or inserted inside said anchoring means 1 by using a thin tape of sponge interposed between the two elements, so as to guarantee an optimum pneumatic sealing of the device 100 during the measurement step.

According to an aspect of the invention, once fastened to the soil and sealed on the upper side by the closing element 10, the main body 5 of the device can be lightly shaken to allow a good mixing of the internal air whenever a measurement is started.

In this regard, it is noted that the height of the main body 5 of the device plays a critical role since, if excessive, it would make that the system takes too long to reach a *"steady state"* regime necessary to determine the moment of recording the measurement; conversely, the humidity variations in the chamber would be too rapid with respect to the response time of the detection means.

According to a preferred embodiment of the invention, the ratio between the height (in mm) and the open lower terminal section (in cm²) of the main body 5 is comprised between 0.77 and 1.74 mm/cm², preferably it is equal to 1.16 mm/cm².

The device according to the invention can also include a fan associated or fixed to the internal surface of the closing element 10 to be facing the inside of the main body 5 when the closing element 10 is coupled to the upper end of the main body 5. The internal ventilation generated by the fan, controlled from outside by a switch included in the device 100 or remotely controlled means, allows that the air in the chamber 101 defined by the main body 5 is always homogeneous in its composition, placing the measurements of the detection means 8 (for example transducers) in optimum equilibrium with the atmosphere humidity variations.

According to an embodiment, the internal surface of the closing element 10 bears or is connected to a supporting element 6, for example in form of a stirrup, configured to house the detection means 8 and/or the fan.

The device according to any one of the herein described embodiments can further comprise a management and control unit operatively associated to the detection means 8, suitable to control the activation/deactivation thereof and to receive the measured data. Preferably, the management and control unit comprises a microprocessor system.

Additionally, the device 100 can comprise at least a storage unit operatively associated to the management and control unit and suitable to store the data measured by the detection means 8, for instance in form of a SD-card.

The device 100 according to any one of the herein described embodiments can further comprise one or more additional electric control means, such as, for example, switch devices operatively connected to said detection means and/or said fan and/or said management and control unit. Such switch devices can include, for example, a first switch 12 configured to activate the fan fastened externally to said closing element 10, as well a start and end data acquisition switch 13 arranged on the external surface of the device.

According to an aspect of the invention, the management and control unit, the storage unit and/or the electric control means can be fastened onto the external surface of the closing element 10 at ad-hoc implemented housings or stirrups. Such components are operatively connected to the detection means arranged in the lower portion of the closing element 10 through gas-tight holes allowing the passage of electrical connections. For example, the electric control means, the management and control unit and/or the storage unit can be placed inside a housing in form of a container fastened on the external upper portion of the closing element 10.

According to a particular aspect of the invention, on the lower surface of the closing element 10, on a stirrup, a fan operating at 12 Volt and sensors of atmospheric pressure, temperature and relative humidity, powered at 5 volt and communicating with a micro-processor of the management and control unit according to the i2c standard, can be installed. By pure way of example, the holes allowing the passage of the electrical connections between the different components of the device can be sealed with hot glue.

According to the invention, the measurements and the electronic recording of the evaporation and/or transpiration parameters from the soil portion under examination, for example the recording of the variations in humidity, temperature, pressure and/or light, start when the main body 5 of the device 100 is closed, by actuating a dedicated switch.

According to an additional aspect of the invention, the device according to any one of the herein described embodiments can further comprise means for transmitting the data measured by said detection means 8, which is associated to the detection means 8 and/or to the management and control unit- The data transmission means can comprise a serial transfer unit, for example a USB port. Moreover, the data transmission means can comprise or be connected to a data displaying unit, for example a display or screen arrange on, or connected to, the external surface of the device 100.

In a preferred embodiment, the device comprises a liquid crystal display as unit for displaying said data, suitable to allow the operator to keep under control the various parameters of the system and to identify possible anomalies both in the electronics itself and in the device chamber such as, for example, the possible lack of sealing of the chamber or humidity values near to saturation.

The device can further include an electronic clock, a thermal sensor for measuring the temperature outside the device and/or a photocell suitable to determine the ambient light, preferably associated to the closing element 10.

Still, the device 100 preferably comprises an autonomous power supply system, for example powered by solar energy.

With particular reference to Figure 4, an embodiment of the device 100 according to the present invention comprises the following commercially available components:
51) an Arduino1 Rev3 micro-controller;
52) an Adafruit shield to protect the data acquisition and storage system (Adafruit industries, NY, USA) provided with a mass memory SD, a clock and a calendar;
53) an OLED 96C 0.96" display (Futura Group s.r.l. divisione elettronica, Gallarate, VA, Italy);
54) a LM335 thermal sensor for measuring T outside the chamber (STMicroelectronics, GE, Switzerland);
55) a BPW20RF silicon photodiode (Vishay Intertechnology Inc., PA, USA) and a 12V 7Ah rechargeable battery (not present in the diagram);
56) a brushless fan (Commonwealth Industrial Corporation, Taiwan);
57) a GY BME280 multi-sensor for measuring temperature, pressure and relative humidity (Bosch Sensortec GmbH, RT, Germany);
58) a Sensirion SCD30 sensor (Sensirion AG, ZH, Switzerland) for additional measurements of CO², temperature and relative humidity.

In an additional embodiment, the device 100 according to the invention comprises a series of logic components as listed hereinafter, with particular reference to the scheme shown in the diagram in Figure 5:
31. an electrical mass of the circuit;
32. a battery powering the circuit, 12-volt nominal tension;
33. a fan switch: when the circuit is closed the fan is actuated;
34. a switch for data recording: when closed, the system records data on mass memory;
35. a main switch: when closed, the electronic component is activated;
36. an ARDUINO1-type microcontroller managing the peripherals of data acquisition, storage and real-time displaying, supports the circuit containing the RTC-type clock and the housing for the mass memory in the form of SD card;
37. SCL and SDA common lines according to 12C standard used for the data exchange between ARDUINO1 and peripherals;
38. a mass and power supply line of peripherals;
39. a backlit liquid crystal display with 16 characters per line for 8 lines;
40. an RTC-type electronic clock with a backup battery composing the ADAFRUIT strip connected through multiple connectors to ARDUINO 1;
41. a casing for the SD card used as mass memory present on the ADAFRUIT card;
42. an environmental data transducer placed inside the measuring chamber: absolute air pressure, temperature and relative humidity;
43. a load resistance of the ambient light sensor placed outside the chamber;
44. an external photo-cell for measuring the ambient light;
45. a temperature sensor outside the chamber;
46. a connector placed on ARDUINO 1 for digital serial communications of USB type;
47. a fan inside the measurement chamber operating with the battery voltage.

In particular, a preferred embodiment according to the present invention relates to a device 100 comprising a main body with cylindrical shape and a closing element with truncated-cone shape on the outer surface thereof, in a plastic container, a micro-processor, an electronic clock, a mass memory in the form of an SD-card, a temperature sensor and a series of connectors used to power and collect data from the various detecting means 8 (transducers) are fastened. On the surface of the said plastic container, laterally, there can be three diverters and, on the upper side, so as to be in horizontal position, a silicon photocell apart from a liquid crystal display, connectors for the battery and means for the electronic communication via USB cable. In the recess of the closing element according to the invention even a pack with three 3.2-nominal Volt lithium batteries can also be placed.

The present invention also relates to a process for measuring the rate of water loss from a portion of bare soil or soil covered by vegetation, comprising the steps of:
- sealingly anchoring to the soil portion a device 100 for measuring the evaporation and/or transpiration parameters according to any one of the previously described embodiments; and
- processing the measurements performed by the device 100 to obtain a measurement of the rate of water loss from said soil portion.

### NOT LIMITING EXAMPLES

### Example 1 - Assembly costs

The following Table 1 shows the overall cost of the components required for assembling a device according to an embodiment of the present invention.

**Table 1**

| | **Costs** | | |
|---|---|---|---|
| **Component** | **(No.)** | **(Euro)** | **(USD)** |
| PVC sheet | 1 | 2.33 | 2.82 |
| Plastic conical structure | 1 | 1.2 | 1.45 |
| Plastic conical lid | 1 | 0.99 | 1.20 |
| Gasket | 1 | 2.5 | 3.03 |
| Steel wire | 1 | 6.9 | 8.35 |
| Battery | 1 | 16 | 19.36 |
| Brushless fan | 1 | 6.77 | 8.19 |
| Arduino1 Rev3 micro-controller | 1 | 22.5 | 27.23 |

| **Component** | **(No.)** | **(Euro)** | **(USD)** |
|---|---|---|---|
| Sensirion SCD30 | 1 | 58.56 | 70.86 |
| GYBMEP BME/BMP280 | 1 | 8.5 | 10.29 |
| Display OLED96C 0.96" | 1 | 11 | 13.31 |
| Adafruit Assembled Data Logging shield for Arduino | 1 | 17.02 | 20.59 |
| Temperature sensor LM335 | 1 | 0.51 | 0.62 |
| Silicon Photodiode BPW20RF | 1 | 6.5 | 7.87 |
| Other electronic material | 14 | 12.8 | 15.49 |
| **Total cost** | | 174.08 | 210.64 |

Due to its dimensional features (volume of 28L for a height of 570 mm and a surface resting onto the soil of 491 cm²), weight (about 920 g) and ease of use, it is estimated that in 1 hour of work at least 20 determinations can be performed. Essential aspect of the invention is that, by associating loT elements, it can be assembled with a total cost of the components ranging around 175 €.

### Example 2 - Operation of the device according to an embodiment of the invention

The measurement of evaporation, if the soil is bare, or of evapotranspiration, if the soil is wholly or partially covered with grass, starts upon closing the device chamber. The monitoring of the increase in humidity of the air contained in the chamber proceeds for the subsequent 120 seconds by updating on display every 10 seconds. The chamber is closed at the bottom by the soil, laterally by the cylinder and on the top by a conical plug, which supports the transducers and, externally, the electronics.

Starting from the curve of increase in humidity inside the chamber it is possible to calculate, by interpolation of a regression function which, derived in its initial portion through suitable algorithm, allows to estimate the water loss in terms of mm per second.

In the following calculation sheet and in the associated Figure 6 the fundamental passages of the calculation starting from the instantaneous data are described.

In particular, Figure 6 shows the curves relating to the evaporation rate (E), t2, and relative humidity (ur2) for a reading performed within 120 seconds with the device according to the invention.

**Calculation of water quantity present in a bell placed on the soil at a given temperature, pressure and air humidity.**

| **Data on 2020_7_22_17_29_17** | | | | | |
|---|---|---|---|---|---|
| **Air temperature...........................** | | in | T (°C) | **31.33** | https://it.wikipedia.org/wiki/Equazione_di_Antoine |
| Antoine constants | | cost | A | 65.81 | |
| Antoine constants | | cost | B | 7066.27 | |
| Antoine constants | | cost | C | 5.976 | |
| Saturated vapor pressure | | out | po, (Pa) | 4573.1091 | exp(A-B/(T+273)-C*In(T+273)) |
| **Relative Humidity.......................** | | in | UR% | **75.01** | |
| Vapor pressure | | out | p, (Pa) | 3430.28913 | po*(UR%/100) |
| **Container height** | | in | h, (cm) | 57 | https://it.wikipedia.org/wiki/Equazione_di_stato |
| **Base area of the container** | | in | s, (cm2) | 490.87 | |
| container volume | | out | V, (liters) | 27.97959 | h*s |
| Gas constant | | cost | R | 8324 | https://it.wikipedia.org/wiki/Costante_dei_gas |
| **Pressure of gases in the container** | | in | Patm (Pa) | **100807** | |
| Number of gas moles | | out | n, (mol) | 1.1128617 | V*Patm/(R*(T+273) |

| **H2O in the chamber at T,Patm, V, UR%** | | | | | |
|---|---|---|---|---|---|
| Number of moles of water | | out | nw (mol) | 0.03786877 | V*p/(R*(T+273) |
| Water mass in the cylinder | | out | E, (gr, ml) | 0.68163791 | nw*18 |
| Equivalent in mm | | out | E, (mm) | 0.01388632 | E/s*10 |

| **Invented data or a series** | | | | | |
|---|---|---|---|---|---|
| t, (sec) | | Pa atm | t2 | ur2 | E, (mm) |
| | 0 | 95832 | 29 | 51.9 | 0.0084 |
| | 15 | 100751 | 31.04 | 51.24 | 0.0093 |
| | 30 | 100754 | 31.24 | 50.58 | 0.0093 |
| | 45 | 100767 | 31.4 | 55.06 | 0.0102 |
| | 60 | 100809 | 31.36 | 64.43 | 0.0119 |
| | 75 | 100808 | 31.32 | 70.4 | 0.013 |
| | 90 | 100807 | 31.33 | 75.01 | 0.0138 |
| | 105 | **100800** | **31.35** | **79.09** | 0.0146 |

The degree of accuracy and stability of the measurements provided by the device according to the invention was initially tested with a laboratory method of gravimetric type. The calibration was performed by determining the evaporation rate (E) from a wet cloth by using both the piece of date recorded by the chamber and the one directly acquired with a precision balance during the processive dehydration process. The weighing system consists of an electronic balance (model PS 2100.R2.M, Radwag, Radom, MZ, Poland) with a resolution equal to 0.01 g. The calibration consisted of 25 weighing cycles each one lasting 120 seconds. For each cycle, the rate of evaporated water in time unit (mm/h) was calculated according to the equations reported in Capri et al, 2021 *[*Capri, C., et al., A low-cost portable chamber based on Arduino micro-controller for measuring cover crops water use. Computers and Electronics in Agriculture, 2021. 190*],* while the gravimetric loss was deduced by weighing the cloth mass before and after measurement with the device of the invention (mm h⁻¹).

### Main results

The obtained interpolation line has the following equation: y = 0.6325x + 0.0856, R² = 0.9611 (Figure 7). The dotted line in figure 7 designates the bisector.

As also visible in Figure 7, the interpolation line characterizes by a very high degree of correlation (R² > 0.96).

However, for progressively increasing values of water loss, there is a slight underestimation of the values obtained gravimetrically by the device of the invention. This result is not surprising since, due to the limited volume of the device according to the invention (28 L), the quick increase in the internal RH determines a quick decrease in the evaporative demand around the wet cloth.

A second calibration test, described with full details by Capri et al., 2021, was performed on 22 July 2020 in an outdoor space adjacent to Piacenza office of the Università Cattolica del Sacro Cuore on vessels having 15 L of volume representing: i) moist soil in surface (WST); ii) dry soil in surface (ST); iii) soil grassed with *Lotus corniculatus* (LC) and soil grassed with *Festuca arundinacea* (FA).

The calibration on one side provided weighing all vessels at the beginning and at the end of the cycle of measurements, comprised between 9:00 and 19:00. Within such interval, evapotranspiration measurements were performed with the device of the invention at intervals of two hours, starting from 9. Data were expressed as daily cumulated water loss (mm d⁻¹).

For each measurement, the values of air temperature and relative humidity (RH) outside and inside the chamber were measured at intervals of 15 seconds and the vapor pressure deficit (VPD) calculated accordingly.

### Main results

The data obtained for *L. corniculatus, F. arundinacea;* wet soil in surface and dry soil in surface are shown in Figure 8.

The obtained interpolation line has the following equation: 1.0649x - 1.5513, R² = 0.9979 (Figure 8). The dotted line in figure 8 designates the bisector.

Despite a slight underestimate of the water loss rate for values lower than 5 mm d⁻¹ by the device of the invention, the measurements performed with the closed chamber showed an excellent correlation with the gravimetric data showing even a minimum deviation with respect the bisector (Figure 8). These results are very encouraging since they confirm that by increasing the measuring time scale (for instance from instantaneous to daily) a consistent reduction in the error, typically accompanying the instantaneous measurement, is obtained.

The present invention has been sofar described with reference to preferred embodiments. It is to be meant that other embodiments belonging to the same inventive core may exist, as defined by the protective scope of the herebelow reported claims.

## Claims

1. A device (100) adapted to measure the evaporation and/or transpiration parameters of a portion of bare soil or soil covered by vegetation, comprising:
▪ a hollow main body (5) extending between an open upper end and an open lower end, opposed to one another, said main body (5) defining a measurement chamber (101);
▪ a closing element (10) adapted to sealingly couple with said upper end, so as to seal on the upper side said main body (5) during the measurement step; said closing element (10) further comprising one or more detection means (8) configured to measure said evaporation and/or transpiration parameters, said detection means (8) being associated with an internal surface of said closing element (10) facing inside the main body (5) when said closing element (10) is coupled with said upper end of the main body (5); and
▪ anchoring means (1) comprising: a proximal terminal end portion configured to implement a sealed coupling with said lower end of the main body (5), and a distal terminal end portion configured to at least partially penetrate the soil portion, wherein said anchoring means (1) is adapted to seal said main body (5) on the lower side by implementing a watertight closure of said measurement chamber, and further adapted to immobilize the device (100) to the soil during the measurement step.

2. The device (100) according to claim 1, wherein said anchoring means (1) comprises an annular body with a truncated conical section bearing a distal terminal end provided with a sharp or tapered outer edge, suitable to penetrate the soil portion.

3. The device (100) according to claims 1 or 2, wherein said anchoring means (1) is provided with manipulation means (2), for example in the form of handles.

4. The device (100) according to any one of the preceding claims, comprising at least one junction element (9) interposed between said closing element (10) and said upper end of the main body (5) and/or at least a junction element (3) interposed between said anchoring means (1) and said lower end of the main body (5), wherein said junction elements (3, 9) preferably consist of a sponge with closed cells.

5. The device (100) according to any one of the preceding claims, wherein said main body (5) has a substantially cylindrical conformation, preferably with constant section.

6. The device (100) according to any one of the preceding claims, wherein said open lower **end** of said main body (5) has a substantially truncated-conical conformation suitable for sealing inserting into said anchoring means (1).

7. The device (100) according to any one of the preceding claims, further comprising a fan associated to said closing element (10) and facing the inside of the main body (5) when said closing element (10) is inserted in the upper end of the main body (5).

8. The device (100) according to any one of the preceding claims, wherein said closing element (10) is provided with a support element (6) suitable for housing said detection means (8) and/or said fan.

9. The device (100) according to any one of the preceding claims, wherein said detection means comprises one or more sensors suitable for measuring said evaporation and/or transpiration parameters in said measurement chamber (101), wherein said parameters comprise at least one of: partial vapor pressure, temperature, relative humidity, carbon dioxide concentration and atmospheric pressure.

10. The device (100) according to any one of the preceding claims, further comprising:
- a management and control unit operatively associated with said detection means (8), suitable for controlling the activation/deactivation of said detection means (8) and for receiving the data measured by said detection means (8); and
- a storage unit operatively associated with said management and control unit and suitable for storing said data measured by said detection means (8).

11. The device (100) according to claim 10, further comprising one or more means for the transmission of said data, associated with said detection means and/or with said management and control unit.

12. The device (100) according to claim 11, wherein said transmission means comprises a **serial** transfer unit and/or a display unit of said data.

13. The device (100) according to any one of the preceding claims, wherein said main body **(5) is** made of a transparent material.

14. The device (100) according to any one of the preceding claims, wherein the ratio between the height and the section of the open lower end of said main body (5) is comprised between 0.77 and 1.74 mm/cm², preferably equal to 1.16 mm/cm².

15. A process for measuring the rate of water loss from a portion of bare soil or soil covered by vegetation, comprising the steps of:
- sealingly anchoring to the soil portion a device (100) for measuring the evaporation and/or transpiration parameters according to any one of claims 1 to 14; and
- processing the measurements performed by the device (100) to obtain a measurement of the rate of water loss from said soil portion.

## Patentansprüche

1. Vorrichtung (100), die angepasst ist, um die Verdampfungs- und/oder Transpirationsparameter eines Abschnitts eines vegetationslosen Erdbodens oder eines durch Vegetation bedeckten Erdbodens zu messen, umfassend:
▪ einen hohlen Hauptkörper (5), der sich zwischen einem offenen oberen Ende und einem offenen unteren Ende erstreckt, die einander gegenüberliegen, wobei der Hauptkörper (5) eine Messkammer (101) definiert;
▪ ein Verschlusselement (10), das angepasst ist, um mit dem oberen Ende abdichtend zu koppeln, um während des Messschritts den Hauptkörper (5) auf der oberen Seite abzudichten; das Verschlusselement (10) ferner umfassend ein oder mehrere Erfassungsmittel (8), die konfiguriert sind, um die Verdampfungs- und/oder Transpirationsparameter zu messen, wobei die Erfassungsmittel (8) mit einer inneren Oberfläche des Verschlusselements (10) verknüpft sind, wobei sie dem Inneren des Hauptkörpers (5) zugewandt sind, wenn das Verschlusselement (10) mit dem oberen Ende des Hauptkörpers (5) gekoppelt ist; und
▪ Verankerungsmittel (1), umfassend: einen proximalen Anschlussendabschnitt, der konfiguriert ist, um ein abgedichtetes Koppeln mit dem unteren Ende des Hauptkörpers (5) auszuführen, und einen distalen Anschlussendabschnitt, der konfiguriert ist, um in den Erdbodenabschnitt mindestens teilweise einzudringen, wobei das Verankerungsmittel (1) angepasst ist, um den Hauptkörper (5) auf der unteren Seite durch Ausführen einer wasserdichten Verschließung der Messkammer abzudichten, und ferner angepasst ist, um die Vorrichtung (100) während des Messschritts an dem Erdboden unbeweglich zu machen.

2. Vorrichtung (100) nach Anspruch 1, wobei das Verankerungsmittel (1) einen ringförmigen Körper mit einem kegelstumpfförmigen Querschnitt umfasst, der ein distales Anschlussende trägt, das mit einer scharfen oder sich verjüngenden Außenkante bereitgestellt ist, die geeignet ist, um in den Erdbodenabschnitt einzudringen.

3. Vorrichtung (100) nach Anspruch 1 oder 2, wobei das Verankerungsmittel (1) mit Manipulationsmitteln (2), zum Beispiel in der Form von Griffen, bereitgestellt ist.

4. Vorrichtung (100) nach einem der vorstehenden Ansprüche, umfassend mindestens ein Verbindungselement (9), das zwischen dem Verschlusselement (10) und dem oberen Ende des Hauptkörpers (5) angeordnet ist, und/oder mindestens ein Verbindungselement (3), das zwischen dem Verankerungsmittel (1) und dem unteren Ende des Hauptkörpers (5) angeordnet ist, wobei die Verbindungselemente (3, 9) vorzugsweise aus einem Schwamm mit geschlossenen Zellen bestehen.

5. Vorrichtung (100) nach einem der vorstehenden Ansprüche, wobei der Hauptkörper (5) eine im Wesentlichen zylindrische Gestalt, vorzugsweise mit konstantem Querschnitt, aufweist.

6. Vorrichtung (100) nach einem der vorstehenden Ansprüche, wobei das offene untere Ende des Hauptkörpers (5) eine im Wesentlichen kegelstumpfförmige Gestalt aufweist, die zum abdichtenden Einsetzen in das Verankerungsmittel (1) geeignet ist.

7. Vorrichtung (100) nach einem der vorstehenden Ansprüche, ferner umfassend einen Lüfter, der mit dem Verschlusselement (10) verknüpft ist und der dem Inneren des Hauptkörpers (5) zugewandt ist, wenn das Verschlusselement (10) in dem oberen Ende des Hauptkörpers (5) eingesetzt ist.

8. Vorrichtung (100) nach einem der vorstehenden Ansprüche, wobei das Verschlusselement (10) mit einem Lagerelement (6) bereitgestellt ist, das zum Aufnehmen der Erfassungsmittel (8) und/oder des Lüfters geeignet ist.

9. Vorrichtung (100) nach einem der vorstehenden Ansprüche, wobei die Erfassungsmittel einen oder mehrere Sensoren umfassen, die zum Messen der Verdampfungs- und/oder Transpirationsparameter in der Messkammer (101) geeignet sind, wobei die Parameter mindestens eines umfassen von:
Partialdampfdruck, Temperatur, relativer Feuchtigkeit, Kohlendioxidkonzentration und atmosphärischem Druck.

10. Vorrichtung (100) nach einem der vorstehenden Ansprüche, ferner umfassend:
- eine mit den Erfassungsmitteln (8) wirkverknüpfte Verwaltungs- und Steuereinheit, die zum Steuern der Aktivierung/Deaktivierung der Erfassungsmittel (8) und zum Empfangen der durch die Erfassungsmittel (8) gemessenen Daten geeignet ist; und
- eine mit der Verwaltungs- und Steuereinheit wirkverknüpfte Speichereinheit, die zum Speichern der durch die Erfassungsmittel (8) gemessenen Daten geeignet ist.

11. Vorrichtung (100) nach Anspruch 10, ferner umfassend ein oder mehrere Mittel für die Übertragung der Daten, die mit den Erfassungsmitteln und/oder mit der Verwaltungs- und Steuereinheit verknüpft sind.

12. Vorrichtung (100) nach Anspruch 11, wobei die Übertragungsmittel eine serielle Transfereinheit und/oder eine Anzeigeeinheit der Daten umfassen.

13. Vorrichtung (100) nach einem der vorstehenden Ansprüche, wobei der Hauptkörper (5) aus einem transparenten Material hergestellt ist.

14. Vorrichtung (100) nach einem der vorstehenden Ansprüche, wobei das Verhältnis zwischen der Höhe und dem Querschnitt des offenen unteren Endes des Hauptkörpers (5) zwischen 0,77 mm/cm² und 1,74 mm/cm² liegt, wobei es vorzugsweise gleich 1,16 mm/cm² ist.

15. Verfahren zum Messen der Wasserverlustrate aus einem Abschnitt von vegetationslosem Erdboden oder durch Vegetation bedecktem Erdboden, umfassend die Schritte:
- abdichtendes Verankern einer Vorrichtung (100) an dem Erdbodenabschnitt zum Messen der Verdampfungs- und/oder Transpirationsparameter nach einem der Ansprüche 1 bis 14; und
- Verarbeiten der durch die Vorrichtung (100) durchgeführten Messungen, um eine Messung der Wasserverlustrate aus dem Erdbodenabschnitt zu erhalten.

## Revendications

1. Dispositif (100) adapté pour mesurer les paramètres d'évaporation et/ou de transpiration d'une partie de sol nu ou de sol recouvert de végétation, comprenant :
▪ un corps principal (5) creux s'étendant entre une extrémité supérieure ouverte et une extrémité inférieure ouverte, opposées l'une à l'autre, ledit corps principal (5) définissant une chambre de mesure (101) ;
▪ un élément de fermeture (10) adapté pour s'accoupler de manière étanche à ladite extrémité supérieure, de manière à sceller sur le côté supérieur ledit corps principal (5) pendant l'étape de mesure ; ledit élément de fermeture (10) comprenant en outre un ou plusieurs moyens de détection (8) configurés pour mesurer lesdits paramètres d'évaporation et/ou de transpiration, lesdits moyens de détection (8) étant associés à une surface interne dudit élément de fermeture (10) orienté vers l'intérieur du corps principal (5) lorsque ledit élément de fermeture (10) est accouplé à ladite extrémité supérieure du corps principal (5) ; et
▪ un moyen d'ancrage (1) comprenant : une partie d'extrémité terminale proximale conçue pour mettre en œuvre un accouplement étanche avec ladite extrémité inférieure du corps principal (5), et une partie d'extrémité terminale distale conçue pour pénétrer au moins partiellement dans la partie de sol, dans lequel ledit moyen d'ancrage (1) est adapté pour sceller ledit corps principal (5) sur le côté inférieur en mettant en œuvre une fermeture étanche à l'eau de ladite chambre de mesure et adapté en outre pour immobiliser le dispositif (100) au sol pendant l'étape de mesure.

2. Dispositif (100) selon la revendication 1, dans lequel ledit moyen d'ancrage (1) comprend un corps annulaire avec une section conique tronquée portant une extrémité terminale distale pourvue d'un bord externe aigu ou effilé, apte à pénétrer dans la partie de sol.

3. Dispositif (100) selon les revendications 1 ou 2, dans lequel ledit moyen d'ancrage (1) est pourvu d'un moyen de manipulation (2), par exemple sous forme de poignées.

4. Dispositif (100) selon l'une quelconque des revendications précédentes, comprenant au moins un élément de jonction (9) interposé entre ledit élément de fermeture (10) et ladite extrémité supérieure du corps principal (5) et/ou au moins un élément de jonction (3) interposé entre ledit moyen d'ancrage (1) et ladite extrémité inférieure du corps principal (5), dans lequel lesdits éléments de jonction (3, 9) consistent de préférence en une éponge à cellules fermées.

5. Dispositif (100) selon l'une quelconque des revendications précédentes, dans lequel ledit corps principal (5) a une conformation sensiblement cylindrique, de préférence de section constante.

6. Dispositif (100) selon l'une quelconque des revendications précédentes, dans lequel ladite extrémité inférieure ouverte dudit corps principal (5) a une conformation sensiblement tronquée-conique appropriée pour une insertion d'étanchéité dans ledit moyen d'ancrage (1).

7. Dispositif (100) selon l'une quelconque des revendications précédentes, comprenant en outre un ventilateur associé audit élément de fermeture (10) et orienté vers l'intérieur du corps principal (5) lorsque ledit élément de fermeture (10) est inséré dans l'extrémité supérieure du corps principal (5).

8. Dispositif (100) selon l'une quelconque des revendications précédentes, dans lequel ledit élément de fermeture (10) est pourvu d'un élément de support (6) apte à loger lesdits moyens de détection (8) et/ou ledit ventilateur.

9. Dispositif (100) selon l'une quelconque des revendications précédentes, dans lequel lesdits moyens de détection comprennent un ou plusieurs capteurs aptes à mesurer lesdits paramètres d'évaporation et/ou de transpiration dans ladite chambre de mesure (101), dans lequel lesdits paramètres comprennent au moins l'une parmi : pression partielle de vapeur, température, humidité relative, concentration de dioxyde de carbone et pression atmosphérique.

10. Dispositif (100) selon l'une quelconque des revendications précédentes, comprenant :
- une unité de gestion et de commande associée de manière fonctionnelle auxdits moyens de détection (8), apte à commander l'activation/la désactivation desdits moyens de détection (8) et à recevoir les données mesurées par lesdits moyens de détection (8) ; et
- une unité de stockage associée de manière fonctionnelle à ladite unité de gestion et de commande et apte à stocker lesdites données mesurées par lesdits moyens de détection (8).

11. Dispositif (100) selon la revendication 10, comprenant en outre un ou plusieurs moyens pour la transmission desdites données, associés auxdits moyens de détection et/ou à ladite unité de gestion et de commande.

12. Dispositif (100) selon la revendication 11, dans lequel lesdits moyens de transmission comprennent une unité de transfert en série et/ou une unité d'affichage desdites données.

13. Dispositif (100) selon l'une quelconque des revendications précédentes, dans lequel ledit corps principal (5) est constitué d'un matériau transparent.

14. Dispositif (100) selon l'une quelconque des revendications précédentes, dans lequel le rapport entre la hauteur et la section de l'extrémité inférieure ouverte dudit corps principal (5) est compris entre 0,77 et 1,74 mm/cm², de préférence égal à 1,16 mm/cm².

15. Procédé permettant de mesurer le taux de perte d'eau d'une partie de sol nu ou de sol recouvert de végétation, comprenant les étapes consistant à :
- ancrer de manière étanche un dispositif (100) à la partie de sol pour mesurer les paramètres d'évaporation et/ou de transpiration selon l'une quelconque des revendications 1 à 14 ; et
- traiter les mesures effectuées par le dispositif (100) pour obtenir une mesure du taux de perte d'eau de ladite partie de sol.
